# EUROPEAN PATENT SPECIFICATION

(11) **EP 2 318 511 B1**
(45) Date of publication and mention of the grant of the patent: **02.08.2017**
(21) Application number: 09782291.0
(22) Date of filing: 27.08.2009
(51) Int. Cl.: C12N 1/04, A23L 33/135

(54) **BACTERIAL COMPOSITION**
BAKTERIELLE ZUSAMMENSETZUNG
COMPOSITION BACTERIÉNNE

(30) Priority: 28.08.2008 DK 200801181; 02.12.2008 EP 08170439
(43) Date of publication of application: 11.05.2011
(73) Proprietor: Chr. Hansen A/S, 2970 Hørsholm (DK)
(72) Inventor: ABRAHAMSEN, Susanne, DK-2960 Karlslunde (DK); STUER-LAURIDSEN, Birgitte, DK-2830 Virum (DK); WINNING, Mette, DK-2860 Soeborg (DK); JOERGENSEN, Lasse Vigel, DK-3500 Vaerløse (DK); JEPPESEN, Iben, DK-3450 Alleroed (DK); WAGNER, Peter, DK-2970 Hoersholm (DK)
(86) International application number: PCT/EP2009/061085
(87) International publication number: WO 2010/023248

(56) References cited:
- WO-A-2008/003781
- WO-A-2008/035332
- WO-A2-00/07571
- DE-C1- 4 109 036
- US-A- 4 806 368
- US-A- 4 927 763
- US-A- 4 956 295
- US-B1- 7 037 708

## Description

### FIELD OF I NVENTI ON

The present invention relates to use of sodium bicarbonate as a color stabilizer in a bacterial composition, wherein the bacterial composition comprises bacterial cells belonging to the *Bifidobacterium animalis* strain DSM15954.

### BACKGROUND OF I NVENTI ON

Several patent documents disclose bacterial formulations which are formulated with specific excipient or carriers which are claimed to increase the storability of the bacterial formulations. Other patent documents relate to lowering the water activity (a_{w}) of the bacterial formulation, e.g. by adding SiO2, which will bind some water. Also use of desiccant in different forms has been used to remove water.

US4956295 discloses dried *Lactobacillii* mixed with a silica gel absorbent. The mixtures are claimed to be storable without refrigeration.

US7122370B and US7229818B disclose formulations comprising probiotic bacteria with monovalent alginate salts, wherein the formulation has a water activity of between 0.01 and 0.07, and wherein, upon exposure to an acidic environment, an alginic acid gel is formed which shields the probiotic bacteria from the antibiotic effects of the acidic environment. The alginate salt is dried to a moisture content below 5% before mixing with the bacteria.

EP1482811 B discloses a process for obtaining a food product comprising the steps of
a. mixing a preparation of viable micro-organisms and further components,
b. drying the mixture to a water activity below 0.3,
c. compacting the mixture
d. coating, and
e. mixing with a food product.

US2005/0100559 discloses a dry bacterial composition having a water activity of less than 0.5.

US6953592B discloses a non-foaming, water-soluble or water-dispersible carbohydrate-based matrix containing entrapped gas in closed pores in an amount which is sufficient to promote dissolution or dispersion of the matrix upon contact with water.

US2005/0266069 claims a viable and stable probiotic formulation for intestinal targeting, comprising: a plurality of probiotic microspheres each comprising: a core comprising one or more probiotic bacteria, a cellulosic excipient, a disintegrant and one or more additives; and an enteric coating capable of being resistant to gastric fluids, having a residual moisture level of less than 5% and a water activity (a_{w}) between 0.1 and 0.5.

WO 2005/063200 discloses a probiotic tablet comprising a probiotic micro-organism (e.g. *Lactobacillus* GG) and other nutritionally active ingredients in two zones; a first zone comprising said probiotic micro-organism and a second zone comprising at least one said other active ingredient. The water activity in the first zone should be no greater than 0.2. It is stated that good viability of the micro-organisms is obtained despite the relatively high overall moisture content.

WO08048731A claims a method for extending the shelf-life of an LGG-containing powdered nutritional formulation by reducing the water activity of the LGG-containing formulation to less than about 0.16 and maintaining the temperature of the formulation at or below 25° C.

US7037708B discloses a composition which comprises at least one *Lactobacillus plantarum* in carrier-bound form, which a) has a particle size of at least about 0.1 mm and b) comprises from about 10E10 to 10E12 cfu/g of at least one microorganism species; c) has a water activity (a_{w}) of less than 0.15 and d) is compressed. It is disclosed that the culture may comprise an effervescent additive.

WO2008/035332 provides methods for preparing compositions of probiotic microorganisms such as *Lactobacillus* and *Bifidobacterium,* comprising the step of microencapsulating live microorganisms to produce a dry formulation and optionally coating the microcapsules, while retaining to a significant extent the viability of the microorganisms.

US4806368 discloses in Example 6 a tablet formulation comprising 15 different bacterial species including *Bifidobacterium bifidus.* There are no bacterial counts related to *Bifidobacterium bifidus.*

WO00/07571 discloses in Example 2.3 and Table 8 that *Bifidobacterium* in a composition comprising carbonate and citric acid ("Mischung 1.3") survive incubation in 0.1 N HCI for 120 m inutes.

However, none of these documents relates to use of sodium bicarbonate as a color stabilizer in a bacterial composition, wherein the bacterial composition comprises bacterial cells belonging to the *Bifidobacterium animalis* strain DSM15954.

A need exists for improved bacterial cells containing compositions having improved color stability. In particular, a need exists for providing color stable compositions comprising the *Bifidobacterial* strain DSM1 5954.

### SUMMARY OF INVENTION

The present inventors have surprisingly found out that a tablet which contains cells of a *Bifidobacterium* strain and a salt of carbonic acid, sodium bicarbonate, has superior stability especially if stored in an environment protecting against exposure to moisture from the surroundings, e.g. in a sealed aluminum bag or closure, or in a closed glass container. The superior stability is most pronounced when the protected tablets are exposed to high temperature over a longer period of time.

Further, the inventors have discovered that addition of a salt of carbonic acid to a composition containing cells of a *Bifidobacterium* strain surprisingly prevents or reduces the discoloration (red-coloring) that normally occurs during storage.

Based on these surprising findings, the discoloration of a composition comprising living cells of a *Bifidobacterium* strain may be reduced or avoided by adding a salt of carbonic acid to the composition, i.e. the color stability of a composition containing bacterial cells belonging to the genus *Bifidobacterium* may be improved by mixing the bacterial cells with a salt of carbonic acid.

Accordingly, the present invention relates to use of sodium carbonate as a color stabilizer in a bacterial composition, wherein the bacterial composition comprises bacterial cells belonging to the *Bifidobacterium animalis* strain DSM15954.

### DETAI LED DISCLOSURE

The improved color stability of a composition containing bacterial cells belonging to the genus *Bifidobacterium* may be obtained by mixing the bacterial cells with a salt of carbonic acid.

The present invention relates to use of sodium carbonate as a color stabilizer in a bacterial composition, wherein the bacterial composition comprises bacterial cells belonging to the *Bifidobacterium animalis* strain DSM15954.

The bacterial cells may be mixed with a carrier before and/or after and/or at the same time as the mixing with the salt. It is presently preferred that all constituents are mixed at the same time.

By compressing the powder, the product will be a bacterial composition in the form of a powder, or a solid form such as a tablet or a pellet.

A salt of carbonic acid can be selected from the group consisting of sodium carbonate, sodium bicarbonate, sodium sesquicarbonate, potassium carbonate, potassium bicarbonate, potassium sesquicarbonate, magnesium carbonate, sodium glycine carbonate, L-lysine carbonate, arginine carbonate, amorphous calcium carbonate, ammonium carbonate, ammonium bicarbonate and combinations thereof. The present invention relates to use of the salt sodium bicarbonate.

The process further may include a drying step, such as freeze-drying, vacuum drying, drying by means of a desiccant, or heating. A drying step may be performed:
i) before the mixing step (i.e. one or more of the bacterial cells or carrier are dried); and/or
ii) after the mixing step (i.e. the mixture is dried); and/or
iii) after the optional compressing step (i.e. the tablet or pellet is dried).

The bacterial cells can be bacterial cells belonging to a genus selected from the group *Bifidobacterium* e.g. a species selected from the group consisting of *Bifidobacterium longum, Bifidobacterium bifidum, Bifidobacterium lactis, Bifidobacterium brevis, Bifidobacterium animalis, Bifidobacterium adolescentis, Bifidobacterium infantis,* such as bacterial cells belonging to a strain selected from the group consisting of *Bifidobacterium animalis* strain DSM15954, *Bifidobacterium longum* subsp. *infantis* strain DSM15953, *Bifidobacterium longum* subsp. *longum* strain DSM15955, and mutants or variants of any of these.

The carrier may further comprise a component selected from the group consisting of: an inorganic acid or a salt thereof, an organic acid or a salt thereof, a carbohydrate, lactose, a sugar alcohol, soluble fibers and starch.

The processes may include further steps, such as a coating step (wherein e.g. a tablet/pellet is coated in a manner known to the skilled person), and/or a packaging step, e.g. comprising placing the powder or the compressed powder (tablet/pellet) in a sealable container, e.g. made of aluminum and/or a polymer.

The process for improving the color stability of a composition containing bacterial cells belonging to the genus *Bifidobacterium* comprises mixing the bacterial cells with a salt of carbonic acid.

The bacterial cells may belong to any of the above mentioned species or strains. The composition may comprise at least 10E5 CFU/mg, such as at least 10E7 CFU/mg or at least 10E9 CFU/mg, CFU being cell form ing units of the bacterial cells.

The invention relates to the use of a salt of carbonic acid, sodium bicarbonate, as a color stabilizer in a bacterial composition comprising bacterial cells belonging to the *Bifidobacterium animalis* strain DSM15954.

### DEFINITIONS

In the present context, the term "tablet" refers to a compressed powder. The term includes all physical forms and all sizes, such as a pill, a pellet, a tablet, etc.

By the term "bacterial composition" should be understood a composition comprising bacterial cells, or a cell culture. The cells are preferably living or dormant, and it is further preferred that the composition contains at least 10E5 cell forming units per gram.

In the present context, the term "mutant" should be understood as a strain derived from a strain of the invention by means of e.g. genetic engineering, radiation and/or chemical treatment. It is preferred that the mutant is a functionally equivalent mutant, e.g. a mutant that has substantially the same, or improved, properties as the mother strain, e.g. as a probiotic. Such a mutant is a part of the present invention. Especially, the term "mutant" refers to a strain obtained by subjecting a strain of the invention to any conventionally used mutagenization treatment including treatment with a chemical mutagen such as ethane methane sulphonate (EMS) or N-methyl-N'-nitro-N-nitroguanidine (NTG), UV light or to a spontaneously occurring mutant.

In the present context, the term "variant" should be understood as a strain which is functionally equivalent to a strain of the invention, e.g. having substantially the same, or improved properties, e.g. as a probiotic. Such variants, which may be identified using appropriate screening techniques, are a part of the present invention.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein, is intended merely to better illuminate the invention and does not pose a limitation on the scope of the invention unless otherwise claimed. No language in the specification should be construed as indicating any non-claimed element as essential to the practice of the invention.

### LEGENDS

Fig 1: Survival of BB-12® in powder H1069 and H1071 when stored at 37 °C for 3 months.
Fig 2: Picture showing the powder blends H1184 A (A), H1184 B (B), H1184 C (C) and H1184 D (D) after two weeks at room temperature.

### EXAMPLES

### Example 1:

Two blends are made, a powder blend H1069 containing grinded BB-12® HA (5.1 %(w/w)and excipients (Mannitol; 10%(w/w), Lactose; 84.4 %(w/w) Magnesium stearate; 0.5 %(w/w) without Sodium bicarbonate, and powder blend H1071 contained grinded BB-12® HA (5.1 %(w/w) and excipients (Mannitol; 10%(w/w), Lactose; 54.4 %(w/w) Magnesium stearate; 0.5 %(w/w) with Sodium bicarbonate; 30%(w/w). The concentration of Sodium bicarbonate is obtained by replacing the lactose part of the excipients with sodium bicarbonate leaving all other components at equal concentrations in the powder.

The powders were packed in aluminum foil bags (20-30 g) and stored at 37°C for 3 months. Bags were taken for analysis of colony forming units (cfu/g) and water activity (aw) just after packaging (0 months), 1 month and 3 month of storage.

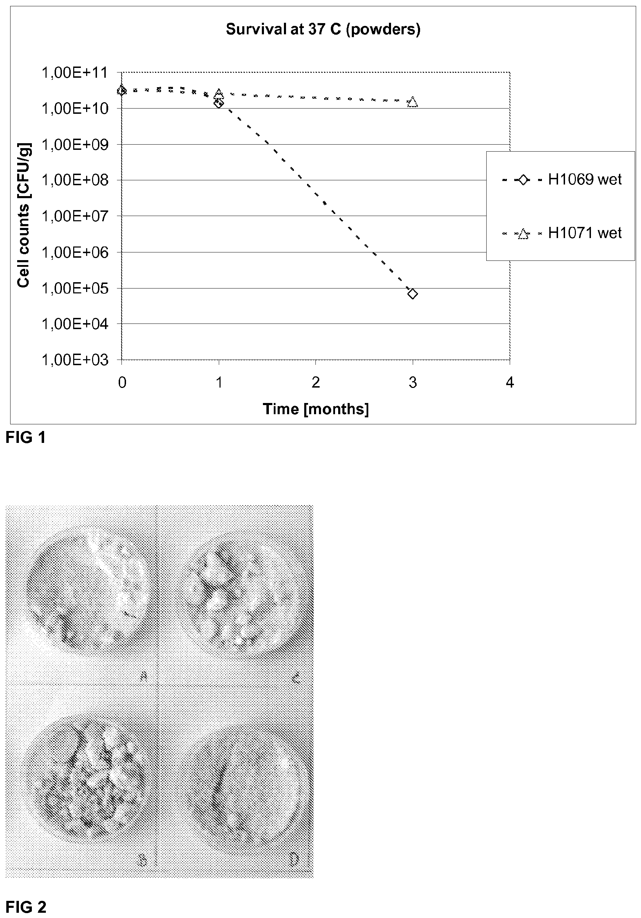

Results: The water activity of the powders H1069 and H1071 was equivalent, aw (H1069) = 0.19 and aw (H1071) = 0.19 just after packaging. After storage for 1 and 3 months the water activity of the powders increased to aw = 0.27-0.31. The results of the colony forming units showed equivalent cfu/g at 0 and 1 month but showed a dramatic difference in survival of BB-12® after 3 months (figure 1).

Conclusion: The replacement of the dextrose excipient with sodium bicarbonate resulted in an improved survival of BB-12® during storage. This result shows that sodium bicarbonate improve survival of *Bifidobacterium animalis* in powders during storage.

### Example 2: Test if sodium bicarbonate will influence the red color development in formulations of Bifidobacterium containing ascorbate.

Contents of powder blends (w/w)
- H1184 A: 24% grinded BB-12® HA culture, 51% mannitol & 25% inulin
- H1184 B: 24% grinded BB-12® HA culture, 51% mannitol & 25% sodium bicarbonate
- H1184 C: 12% grinded BB-12® HA culture, 63% mannitol & 25% inulin
- H1184 D: 12% grinded BB-12® HA culture, 63% mannitol & 25% sodium bicarbonate

The grinded BB-12® HA culture contains ascorbate as a cryoprotective agent. The ascorbate reacts with residues from the fermentation and develops a red color. The reaction is accelerated by elevated temperatures and humidity.

The powders were kept in air-tight aluminum foil bags. For the testing the powders were taken out of the alu bags in amounts of 5-8 g and placed in small plastic containers (manufactured by Rotronic for use in aw measurements). The plastic containers were left at room temperature and after two weeks the difference in color development could be seen as shown in figure 2. The powders had a similar aw of approx. 0.4. Figure 2 shows that the A and C powders have changed color to pale red, while the B and D powders have kept their original pale yellow color.

Conclusion: The presence of sodium bicarbonate in the powder blends has an effect on the development of red color. The red color does not develop when sodium bicarbonate is present in the powder blend.

### REFERENCES

US2004175389, WO03075676, US2005100559, US6953592, WO03012819, WO05266069, WO05063200

## Claims

1. Use of sodium bicarbonate as a color stabilizer in a bacterial composition, wherein the bacterial composition comprises bacterial cells belonging to the *Bifidobacterium animalis* strain DSM15954.

2. Use according to claim 1 wherein the bacterial composition is in the form of a powder, or a solid form such as a tablet or a pellet.

3. Use according to claim 1 or 2 wherein the composition comprises at least 10E5 CFU/mg, such as at least 10E7 CFU/mg or at least 10E9 CFU/mg.

## Patentansprüche

1. Verwendung von Natriumbicarbonat als Farbenstabilisator in einer bakteriellen Zusammensetzung, wobei die bakterielle Zusammensetzung Bakterienzellen umfasst, die dem *Bifidobacterium-animalis-*Stamm DSM15954 angehören.

2. Verwendung nach Anspruch 1, wobei die bakterielle Zusammensetzung die Form eines Pulvers hat oder eine feste Form wie zum Beispiel eine Tablette oder ein Pellet.

3. Verwendung nach Anspruch 1 oder 2, wobei die Zusammensetzung mindestens 10E5 CFU/mg umfasst, wie zum Beispiel mindestens 10E7 CFU/mg oder mindestens 10E9 CFU/mg.

## Revendications

1. Utilisation de bicarbonate de sodium en tant que stabilisant de couleur dans une composition bactérienne, où la composition bactérienne comprend des cellules bactériennes appartenant à la souche *Bifîdobacterium animalis* DSM15954.

2. Utilisation selon la revendication 1 où la composition bactérienne est sous forme d'une poudre, ou sous forme solide comme une tablette ou une pastille.

3. Utilisation selon la revendication 1 ou 2 où la composition comprend au moins 10E5 UFC/mg, comme au moins 10E7 UFC/mg ou au moins 10E9 UFC/mg.
